(19)

**Europäisches Patentamt**
**European Patent Office**
**Office européen des brevets**

(11)  **EP 4 684 774 A1**

(12)  **EUROPEAN PATENT APPLICATION**

(43) Date of publication:
**28.01.2026  Bulletin 2026/05**

(51) International Patent Classification (IPC):
**A61K 8/27** (2006.01)    **A61K 8/29** (2006.01)
**A61K 8/49** (2006.01)    **A61K 8/81** (2006.01)
**A61Q 17/04** (2006.01)

(21) Application number: **25191654.0**

(22) Date of filing: **24.07.2025**

(52) Cooperative Patent Classification (CPC):
**A61K 8/29; A61K 8/27; A61K 8/8182; A61Q 17/04**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority:  **24.07.2024  US 202418782894**

(71) Applicant: **Kenvue Brands LLC**
**Summit, NJ 07901 (US)**

(72) Inventors:
• **LUTS, Tatiana**
  **Summit, 07901 (US)**
• **LONG, Elizabeth**
  **Summit, 07901 (US)**
• **KELLER, Karlye**
  **Summit, 07901 (US)**

(74) Representative: **Carpmaels & Ransford LLP**
**One Southampton Row**
**London WC1B 5HA (GB)**

(54)  **MINERAL SUNSCREEN CONTAINING ZINC OXIDE AND TITANIUM DIOXIDE**

(57)  The present invention provides a sunscreen composition comprising: (i) $TiO_2$, (ii) ZnO, (iii) triacontanyl PVP, and (iv) VP/hexadecene copolymer, wherein the composition is an oil-in-water emulsion substantially free of low molecular weight, organic UV absorbers, the ratio of (iii):(iv) is less than one, and the composition provides an in vitro SPF as measured by the IN VITRO SPF TEST METHOD of at least 25. The composition is surprisingly low viscosity and provides a high SPF.

**Description**

The present invention provides a sunscreen composition comprising: (i) $TiO_2$, (ii) ZnO, (iii) triacontanyl PVP, and (iv) VP/hexadecene copolymer, wherein the composition is an oil-in-water emulsion substantially free of low molecular weight, organic UV absorbers, the weight ratio of (iii):(iv) is less than one, and the composition provides an in vitro SPF as measured by the IN VITRO SPF TEST METHOD of at least 25. The composition is surprisingly low viscosity and provides a high SPF.

BACKGROUND OF THE INVENTION

It is well known that prolonged exposure to ultraviolet radiation, such as from the sun, can lead to the formation of light dermatoses and erythemas, and increase the risk of skin cancers, such as melanoma. Exposure to UV radiation also accelerates skin aging, such as loss of skin elasticity and wrinkling.

For these reasons, sunscreen compositions are commonly used to provide protection from the sun, and a variety of sunscreen compositions are commercially available. Many contain organic UV absorbers such as octocrylene, homosalate, avobenzone, oxybenzone, octyl salicylate and cinnamates. While these UV filters are effective for absorbing UV radiation, high amounts are often required to provide a high SPF sunscreen composition. Because they are organic compounds, consumers are sometimes concerned about their potential to penetrate the skin, particularly when used in high amounts.

As an alternative, mineral-based sunscreens are also commercially available. These typically contain $TiO_2$, ZnO, or both. For example, AVEENO Calm + Restore Daily Moisturizer Mineral Sunscreen SPF 30, commercially available from Johnson & Johnson Consumer Inc., contains 5.8% by weight $TiO_2$, 12.5% by weight ZnO, 1.5% by weight triacontanyl PVP, and 1% by weight VP/hexadecene copolymer.

Commonly assigned US Patent Application No. 18/136456 filed April 19, 2023, relates to an anhydrous sunscreen composition comprising $TiO_2$ and ZnO. The anhydrous composition is preferably sprayable, and comprises: (a) at least about 15% by weight based on the total weight of the sunscreen composition of a combination of $TiO_2$ and ZnO, wherein the weight ratio of ZnO to $TiO_2$ in the composition is about 0.5 to about 1.5; (b) at least about 50 % by weight based on the total weight of the sunscreen composition of a carrier oil, wherein the carrier oil comprises at least 50% by weight helianthus annuus (sunflower) seed oil; (c) about 10% to about 20% by weight based on the total weight of the sunscreen composition a combination of isododecane and diisopropyl adipate; and (d) about 2% to about 6% by weight based on the total weight of the sunscreen composition of a film former comprising triacontanyl PVP. The '456 application discloses the optional use of a combination of triacontanyl PVP and VP/hexadecene copolymer, and in one embodiment, the composition comprises about 2.5% to about 3.2% by weight of triacontanyl PVP and about 2.5% by weight of VP/hexadecene copolymer. Example F of the '456 application is a comparative anhydrous composition containing 15% by weight $TiO_2$, 10% by weight ZnO, 1.5% by weight triacontanyl PVP, and 2.5% by weight VP/hexadecene copolymer. Example F was unstable; sedimentation at room temperature occurred.

Typically, mineral-only sunscreens are not capable of providing the highest SPF levels without increasing the amounts of mineral UV active ingredients to high levels, which in turn requires a high viscosity formulation to suspend the UV-blockers, i.e., $TiO_2$ and ZnO. For example, there are very few SPF 70 mineral sunscreens on the market today that also have the desirable aesthetics of a low viscosity liquid.

Applicants have now discovered high SPF, oil-in-water emulsions comprising only mineral UV active ingredients that also contain triacontanyl PVP and VP/hexadecene copolymer, wherein the weight ratio of triacontanyl PVP to VP/hexadecane copolymer is less than one. These compositions advantageously provide in vitro SPF levels of at least about 25, and in vivo SPF levels of at least 50, preferably at least 70. As emulsions, they also have excellent liquid aesthetics as a result of surprisingly low viscosity.

SUMMARY OF THE INVENTION

The present invention provides a sunscreen composition comprising: (i) $TiO_2$, (ii) ZnO, (iii) triacontanyl PVP, and (iv) VP/hexadecene copolymer, wherein the composition is an oil-in-water emulsion substantially free of low molecular weight, organic UV absorbers, the weight ratio of (iii):(iv) is less than one, and the composition provides an in vitro SPF as measured by the IN VITRO SPF TEST METHOD of at least 25.

DETAILED DESCRIPTION OF THE INVENTION

Unless defined otherwise, all technical and scientific terms used herein have the same meaning as commonly understood by one of ordinary skill in the art to which the invention belongs. All publications, patent applications, patents, and other references mentioned herein are incorporated by reference.

As used herein, "topically applying" means directly laying on or spreading on outer skin or he scalp, e.g., by use of the hands or an applicator such as a wipe, roller, or spray.

As used herein, "cosmetic" refers to a beautifying substance or preparation which preserves, restores, bestows, simulates, or enhances the appearance of bodily beauty or appears to enhance the beauty or youthfulness, specifically as it relates to the appearance of tissue or skin.

As used herein, "sunscreen composition" means a formulation (e.g., a lotion, spray, gel or other topical product) that absorbs and/or reflects some of the sun's ultraviolet (UV) radiation and thus helps protect against negative effects of sun exposure, e.g., sunburn, premature aging, etc.

As used herein, "cosmetically effective amount" means an amount of a physiologically active compound or composition sufficient for treating one or more conditions, but low enough to avoid serious side effects. The cosmetically effective amount of the compound or composition will vary with the condition being treated, the age and physical condition of the end user, the severity of the condition being treated/prevented, the duration of the treatment, the nature of other treatments, the specific compound or product/composition employed, the cosmetically-acceptable carrier utilized, and like factors.

As used herein, "cosmetically acceptable" means that the ingredients the term describes are suitable for use in contact with tissues (e.g., the skin) without undue toxicity, incompatibility, instability, irritation, allergic response, or the like.

As used herein, a "cosmetically acceptable active agent" is a compound (synthetic or natural) that has a cosmetic or therapeutic effect on the skin.

As used herein, "treatment or treating" refers to mitigating, reducing, preventing, improving, or eliminating the presence or signs of a condition or disorder.

As used herein, "substantially free of" means the ingredient referred to is not directly or intentionally added. Preferably, "substantially free of" means containing less than about 1% by weight. More preferably "substantially free of" means containing less than about 0.5% by weight. Even more preferably "substantially free of" means containing less than about 0.1% by weight. The present sunscreen composition may be completely free of an ingredient, i.e., contain none of the ingredient.

As used herein, "UV absorber" means an active ingredient whose specific function is to absorb radiation in some portion of the ultraviolet spectrum (290nm-400nm), such as one having an extinction coefficient of at least about 1000 $mol^{-1}\ cm^{-1}$, for at least one wavelength within the above-defined ultraviolet spectrum. UV absorbers include for example approved sun filters and are distinguished from compounds having other functions that also happen to absorb UV radiation.

As used herein, "UV blocker" means a compound that reflects or scatters UV radiation.

As used herein, "phase-stable" means the water and oil phases of an emulsion do not appreciably separate at room temperature for at least two weeks.

Unless otherwise indicated, a percentage or concentration refers to a percentage or concentration by weight (i.e., % (W/W) based on the total weight of the composition. Unless stated otherwise, all ranges are inclusive of the endpoints, e.g., "from 4 to 9" includes the endpoints 4 and 9.

MINERAL UV ACTIVE INGREDIENTS

The sunscreen composition of the invention comprises a combination of mineral UV active ingredients, specifically titanium dioxide ($TiO_2$) and zinc oxide (ZnO).

The composition comprises about 9.0 to about 32.0% by weight of a combination of $TiO_2$ and ZnO based on the total weight of the composition. Preferably, the composition comprises about 12.0 to about 28.0% by weight of a combination of $TiO_2$ and ZnO based on the total weight of the composition.

For example, the composition may comprise about 23% by weight of a combination of $TiO_2$ and ZnO based on the total weight of the sunscreen composition.

The weight ratio of zinc oxide to titanium dioxide in the composition ranges from about 3:1 to about 1:1.

In one embodiment, the composition comprises about 7% by weight $TiO_2$ and/or about 14% by weight ZnO. In one embodiment, the composition comprises about 6.64% by weight $TiO_2$ and/or about 14.4% by weight ZnO. In one embodiment, the composition comprises about 6.64% by weight $TiO_2$ and about 14.4% by weight ZnO.

An example of titanium dioxide that may be used is Titanium Dioxide; Aluminum Hydroxide; Stearic Acid sold under the trade name, MICRO TITANIUM DIOXIDE MT-I00TV by Tayca Corporation.

A Titanium Dioxide; Aluminum Hydroxide; Stearic Acid component may contain about 83% titanium dioxide. Thus, in one embodiment, the composition comprises about 8% by weight of Titanium Dioxide; Aluminum Hydroxide; Stearic Acid, and hence comprises about 6.6% by weight of Titanium Dioxide.

An example of zinc oxide that may be used is Zinc Oxide; Triethoxycaprylylsilane sold under the trade name, Micro Zinc Oxide MZX-304OTS by Tayca Corporation.

A Zinc Oxide; Triethoxycaprylylsilane component may contain about 96% of zinc oxide. Thus, in one embodiment, the composition comprises about 15% by weight of Zinc Oxide; Triethoxycaprylylsilane, and hence comprises about 14.4% by weight of Zinc Oxide.

Preferably, the composition is substantially free of UV absorbers. In particular, the composition may be substantially free of low molecular weight, organic UV absorbers. As used herein, "low molecular weight, organic UV absorber" means a UV absorber that is an organic molecule, typically an aromatic compound conjugated with a carbonyl moiety substituted in the ortho- or para- position of the aromatic ring and having a molecular weight of less than 900 g/mol.

Preferably, the composition is completely free of low molecular weight, organic UV absorbers.

Examples of low molecular weight, organic UV absorbers include, but are not limited to: methoxycinnamate derivatives such as octyl methoxycinnamate and isoamyl methoxycinnamate; camphor derivatives such as 4-methyl benzylidene camphor, camphor benzalkonium methosulfate, and terephthalylidene dicamphor sulfonic acid; salicylate derivatives such as octyl salicylate, ethylhexyl salicylate and homosalate; benzone derivatives such as dioxybenzone, and oxybenzone; benzoic acid derivatives such as aminobenzoic acid and octyldimethyl para-amino benzoic acid; octocrylene and other $\beta,\beta$-diphenylacrylates; dioctyl butamido triazone; octyl triazone; avobenzone (butyl methoxydibenzoylmethane);menthyl anthranilate; triazone derivatives such as ethylhexyl triazone (Uvinul® T150); diethylhexyl butamido triazone (UVASorb® HEB); bis-ethylhexyloxyphenol methoxyphenyl triazine (Tinosorb® S), benzoate derivatives such as diethylamino hydroxybenzoyl hexyl benzoate (Uvinul® A Plus), benzotriazole derivatives such as drometrizole trisiloxane (Mexoryl® XL), methylene bis-benzotriazolyl tetramethylbutylphenol (Tinosorb® M); tris-biphenyl triazine; (2-{4-[2-(4-Diethylamino-2-hydroxy-benzoyl)-benzoyl]-piperazine-1-carbonyl}-phenyl)-(4-diethylamino-2-hydroxy-phenyl)-methanone; merocyanine derivatives; bis(butylbenzoate) diaminotriazine aminopropylsiloxane; and bis-ethylhexyloxyphenol methoxyphenyl triazine encapsulated in a polymer matrix.

The composition may in certain embodiments also be free of polymeric organic UV absorbers. As used herein, "polymeric organic UV absorber" means a polymeric compound comprising organic chromophores attached to a polymer chain. For instance, the polymer chain may be a polysiloxane having for example an average molecular weight of greater than 6000 Daltons. Examples of such polysiloxane UV absorbers include, without limitation, Parsol® SLX commercially available from DSM and polysilicone-15. These polysiloxanes absorb in the UVB ($\lambda_{max}$ = 312 nm) part of the spectrum and are typically combined with UVA filters to achieve broad-spectrum protection.

Alternatively, the composition may contain one or more polymeric organic UV absorbers. Because of their relatively high molecular weight, they are regarded as substantially non-absorbing to the skin.

FILM FORMER

The composition also comprises the two film formers triacontanyl PVP (tricontanyl povidone or 2-Pyrrolidinone, 1-ethenyl-, polymer with 1-triacontene) and VP/hexadecene copolymer (vinylpyrrolidone/hexadecene copolymer).

The composition typically comprises about 0.5 to about 2.25 % by weight of triacontanyl PVP, preferably about 0.8 to about 1.5% by weight of triacontanyl PVP. For example, the composition comprises about 1% (such as about 1.0%) by weight of triacontanyl PVP.

The composition typically comprises about 0.5 to about 3% by weight of VP/hexadecene copolymer, preferably about 1 to about 2.5% by weight of VP/hexadecene copolymer. For example, the composition comprises about 2% (such as about 2.0%) by weight of VP/hexadecene copolymer.

Importantly, to achieve high SPF levels for the composition, the weight ratio of triacontanyl PVP to VP/hexadecene copolymer must be less than one. For example, the weight ratio of triacontanyl PVP to VP/hexadecene copolymer may be around 0.5. For example, the weight ratio of triacontanyl PVP to VP/hexadecene copolymer may be 0.5.

In one embodiment, the composition comprises about 1% by weight triacontanyl PVP and about 2% by weight of VP/hexadecene copolymer.

Triacontanyl PVP (tricontanyl povidone or 2-Pyrrolidinone, 1-ethenyl-, polymer with 1-triacontene) is commercially available for example from ISP Freetown Fine Chemicals Inc. under the trade name Antaron™ WP-660 polymer.

VP/hexadecene copolymer is also commercially available for example from ISP Freetown Fine Chemicals Inc. under the trade name Antaron™ V216 polymer.

SPF

Sun protection factor (SPF) of the composition may be tested using the following IN VITRO SPF TEST METHOD. The baseline transmission of a PMMA plate (substrate, available from Helioscience, Marseille, France) is measured for UV absorbance using calibrated Labsphere® UV-1 0005 UV transmission analyzer or a Labsphere® UV-2000S UV transmission analyzer (Labsphere, North Sutton, N.H., USA). A test sample is then applied to the PMMA plate using an application density of about 0.75 mg/cm2 by rubbing into a uniform thin layer with the operator's finger. The sample is allowed to dry for 15 minutes and then measured for UV absorbance in the same way. The absorbance measures are used to calculate SPF as known in the art using the following equation:

$$SPF\ in\ vitro = \frac{\int_{\lambda=290\ nm}^{\lambda=400\ nm} E(\lambda)*I(\lambda)*d\lambda}{\int_{\lambda=290\ nm}^{\lambda=400\ nm} E(\lambda)*I(\lambda)*10^{A_0(\lambda)}*d\lambda}$$

in which:

E($\lambda$) = Erythema action spectrum;
I($\lambda$) = Spectral irradiance received from the UV source;
$A_0(\lambda)$ = Mean monochromatic absorbance of the test product layer before UV exposure; and
d($\lambda$) = Wavelength step (1 nm).

In one embodiment, the composition has an SPF as measured by the IN VITRO SPF TEST METHOD of at least about 25. In another embodiment, the composition has an SPF as measured by the IN VITRO SPF TEST METHOD of at least about 30.

In one embodiment, the composition has an in vivo SPF level of at least 50, preferably at least 70.

SHEAR VISCOSITY TEST METHOD

The composition advantageously has a low shear viscosity. Shear viscosity is measured by the following method. An appropriate amount of sample is loaded into an Anton Paar MCR302 rheometer equipped with a doublewall Couette geometry. Temperature is held at 25° C by a Peltier 5 element, and the shear rate is ramped from 1,000 to 0.1 s-1 using the default steady-state criteria in the rheometer control software RheoCompass.

The composition preferably has a shear viscosity of less than 32 Pa·s at 1 s$^{-1}$ at 25° C. For example, the composition may have a shear viscosity of less than 28 Pa·s at 1 s$^{-1}$ at 25° C.

TOPICAL COMPOSITION

The sunscreen composition can be used by topically applying to a mammal, e.g., by the direct laying on, wiping or spreading of the composition on the skin, hair, or nails of a mammal, particularly a human.

The sunscreen composition is in the form of an oil-in-water emulsion containing a continuous water phase and a discontinuous oil phase dispersed within the continuous water phase. The composition is preferably phase stable.

The oil phase may be such that it is present in discrete droplets or units having an average diameter of about one micron to about 1000 microns, such as from about 1 micron to about 100 microns.

The percentage by weight of water phase included in the compositions may range from about 27.1% to about 85.95%, such as from about 40% to about 50%.

The sunscreen composition may be prepared using mixing and blending methodology well known in the sunscreen and cosmetic art. Typically, the sunscreen composition is produced by preparing an oil phase containing the oil soluble and oil-miscible ingredients, for example caprylic/capric triglyceride, ethylhexyl methoxycrylene, dimethicone, polyhydroxystearic acid, titanium dioxide, aluminum hydroxide, stearic acid, zinc oxide, triethoxycaprylylsilane, cetearyl olivate, sorbitan olivate, calcium sodium borosilicate, steareth-21, triacontanyl PVP, and butyloctyl salicylate; and preparing a water phase by mixing water with the water-soluble or water-miscible ingredients, for instance xanthan gum, disodium EDTA, sodium chloride, chlorphenesin, caprylyl glycol, phenoxyethanol, ethylhexylglycerin, glycerin, and tocopheryl acetate. The oil phase and the water phase may then be mixed in a manner sufficient to disperse the oil phase substantially homogeneously in the water phase such that the water phase is continuous and the oil phase discontinuous.

Applicants have found that optimum dispersion of the $TiO_2$ in the composition is a driver of high SPF. To increase dispersion of the $TiO_2$ it is added at room temperature (i.e., without heating) before the ZnO is added.

The composition may be combined with a "cosmetically-acceptable topical carrier," i.e., a carrier for topical use that capable of containing the other ingredients dispersed or dissolved therein and possessing acceptable properties rendering it safe to use topically.

The composition may optionally comprise a wide variety of additional oil-soluble materials and/or water-soluble materials conventionally used in compositions for use on skin, at their art-established levels. For example, surfactants, pearlescent or opacifying agents, thickeners, emollients, conditioners, humectants, chelating agents, exfoliants, and additives that enhance the appearance, feel, or fragrance of the cleansing composition, such as colorants, fragrances, preservatives, pH adjusting agents, and the like, can be included, provided they do not interfere with the high SPF and low viscosity of the composition.

In one embodiment the composition is substantially free or, or free of, waxes, structuring agents, and fatty alcohols.

Water soluble or water dispersible polymers may be added to the compositions. The water dispersible polymers are comprised of a water-insoluble polymer that is typically micronized and dispersed into a water carrier, possibly with the use of a surface active dispersing aid. The water dispersible polymers are capable of forming a film and improving water resistance of the compositions. Examples of water-soluble polymers include Polyaldo® 10-1-L (Polyglyceryl-10 Laurate), available from Lonza. Examples of water dispersible polymers include water dispersible polyurethanes, such as Baycusan® C1000 (Polyurethane-34), available from Bayer, Dow Corning® 2501 (Bis-PEG-18 Methyl Ether Dimethyl Silane), available from Dow Corning, Eastman AQTM 38S (Polyester-5), available from Eastman Chemical, and Intelimer® 8600 (C8-22 Alkyl Acrylates/Methacrylic Acid Crosspolymer) available from Air Products.

The sunscreen composition may also comprise certain additional film formers, provided they do not interfere with the SPF and low viscosity of the composition. Acceptable additional film formers include polyethylene, which is available from New Phase Technologies as Performalene® 400, a polyethylene having a molecular weight of 400. Another suitable film former is polyethylene 2000 (molecular weight of 2000), which is available from New Phase Technologies as Performalene®. Another suitable film former is synthetic wax, also available from New Phase Technologies as Performa® V-825. Other typical film-formers include acrylates/acrylamide copolymer, acrylates copolymer, acrylates/C12-C22 alkylmethacrylate copolymer, polyethylene, waxes, VP/dimethiconylacrylate/polycarbamylpolyglycol ester, butylated PVP, PVP/hexadecene copolymer, octadecene/MA copolymer, PVP/eicosene copolymer, tricontanyl PVP, Brassica Campestris/Aleuritis Fordi Oil copolymer, decamethyl cyclopentasiloxane (and) trimethylsiloxysilicate, Dimethicone; Acrylates/Dimethicone Copolymer and mixtures thereof. In some cases, the film former is acrylates/C12-C22 alkylmethacrylate copolymer sold under the tradename Allianz™ OPT by Ashland.

Suitable emollients for use in the composition include mineral oils, petrolatum, vegetable oils (e.g. triglycerides such as caprylic/capric triglyceride, Helianthus Annuus (Sunflower) Seed Oil), waxes and other mixtures of fatty esters, including but not limited to esters of glycerol (e.g, isopropyl palmitate, isopropyl myristate, diisopropyl adipate, dicaprylyl carbonate, c12-15 alkyl benzoate, isononyl isononanoate, neopentyl glycol diheptonoate, neopentyl glycol dibenzoate, butyloctyl salicylate, ethylhexylmethoxycrylene), hydrocarbons (e.g, isododecane, isohexadecane, squalane), ethers (e.g, PPG-15 stearyl ether, dicaprylyl ether), and silicone oils such as dimethicone. In certain embodiments, mixtures of triglycerides (e.g. caprylic/capric triclycerides) and esters of glycols (e.g. isopropyl myristate).

The sunscreen composition may comprise one or more emulsifiers. In one embodiment, the sunscreen composition comprises steareth-21. Preferably, the sunscreen composition comprises steareth-21, cetearyl olivate and sorbitan olivate. The ingredient Cetearyl Olivate; Sorbitan Olivate is commercially available as Olivem 1000 from Hallstar.

Examples of suitable solvents include propylene glycol, polyethylene glycol, polypropylene glycol, glycerol, 1,2,4-butanetriol, sorbitol esters, 1,2,6-hexanetriol, ethanol, and mixtures thereof.

In certain embodiments, the composition includes a pigment suitable for providing color or hiding power. The pigment may be one suitable for use in a color cosmetic product, including compositions for application to the hair, nails and/or skin, especially the face. Color cosmetic compositions include, but are not limited to, foundations, concealers, primers, blush, mascara, eyeshadow, eyeliner, lipstick, nail polish and tinted moisturizers. The pigment suitable for providing color or hiding power may be composed of iron oxides, including red and yellow iron oxides, titanium dioxide, ultramarine and chromium or chromium hydroxide colors, and mixtures thereof. The pigment may be a lake pigment, e.g. an organic dye such as azo, indigoid, triphenylmethane, anthraquinone, and xanthine dyes that are designated as D&C and FD&C blues, browns, greens, oranges, reds, yellows, etc., precipitated onto inert binders such as insoluble salts. Examples of lake pigments include Red #6, Red #7, Yellow #5, Violet #2 and Blue #1. The pigment may be an interference pigment. Examples of interference pigments include those containing mica substrates, bismuth oxycloride substrates, and silica substrates, for instance mica/bismuth oxychloride/iron oxide pigments commercially available as CHROMALITE pigments (BASF), titanium dioxide and/or iron oxides coated onto mica such as commercially available FLAMENCO pigments (BASF), mica/titanium dioxide/iron oxide pigments including commercially available KTZ pigments (Kobo products), CELLINI pearl pigments (BASF), and borosilicate-containing pigments such as REFLECKS pigments (BASF).

In one embodiment, the sunscreen composition comprises a humectant. Examples of humectants include, without limitation to, glycerin, pentylene glycol, butylene glycol. In another embodiment, the sunscreen composition comprises at least 3 weight percent glycerin. The sunscreen composition may comprise for example about 5 weight percent glycerin.

In another embodiment, the sunscreen composition has a pH of about 7.0 to about 8.9. The sunscreen composition may have a pH of about 8.0.

In a further embodiment, the sunscreen composition comprises hyaluronic acid. In another embodiment, the sunscreen composition comprises at least 0.01 weight percent hyaluronic acid.

The composition may further comprise one or more other cosmetically acceptable active agents include for example anti-acne agents, shine control agents, anti-microbial agents, anti-inflammatory agents, anti-mycotic agents, anti-parasite agents, external analgesics, antioxidants, keratolytic agents, moisturizers, nutrients, vitamins, energy enhancers, anti-perspiration agents, astringents, deodorants, firming agents, anti-callous agents, and agents for skin conditioning.

The amount of other cosmetically active agents may range from about 0.001% to about 20% by weight of the composition, e.g., about 0.005% to about 10% by weight of the composition, such as about 0.01% to about 5% by weight of the composition.

The cosmetically acceptable active agent may be selected for instance from D-panthenol carotenoids, ceramides, polyunsaturated fatty acids, essential fatty acids, enzymes such as laccase, enzyme inhibitors, minerals, steroids such as hydrocortisone, 2-dimethylaminoethanol, copper salts such as copper chloride, peptides like argireline, syn-ake, acetyl dipeptide-31 amide and those containing copper, coenzyme Q10, amino acids such as proline, vitamins, lactobionic acid, acetyl-coenzyme A, niacin, riboflavin, thiamin, ribose, electron transporters such as NADH and FADH2, natural extracts such as from aloe vera, feverfew, oatmeal, dill, blackberry, princess tree, *Picia anomala,* and chicory, resorcinols such as 4-hexyl resorcinol, curcuminoids, sugar amines such as N-acetyl glucosamines, and derivatives and mixtures thereof.

Examples of vitamins and their derivatives include, but are not limited to, vitamin A, vitamin B's such as vitamin B3, niacinamide, vitamin B5, and vitamin B12, vitamin C, vitamin K, and different forms of vitamin E such as alpha, beta, gamma or delta tocopherols or their mixtures, and derivatives thereof.

Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), tocopherols (e.g., tocopherol acetate), tocotrienols, and ubiquinone. Natural extracts containing antioxidants suitable for use in the compositions of this invention, include, but not limited to, extracts containing flavonoids and isoflavonoids and their derivatives (e.g., genistein and diadzein), extracts containing resveratrol and the like. Examples of such natural extracts include grape seed, green tea, pine bark, propolis and Zingiber Officinale (Ginger) Root Extract.

In one embodiment, the composition comprises one or both of tocopherol and feverfew extract (for example Chrysanthemum Parthenium (Feverfew) Flower/Leaf/Stem Juice).

In one embodiment, the composition comprises phenoxyethanol, chlorphenesin, ethylhexylglycerin and caprylyl glycol. This specific combination of preservatives surprisingly increases the shelf life of compositions according to the invention. Preferably, the composition comprises about 0.6 % by weight phenoxyethanol, about 0.27% by weight chlorphenesin, about 0.5% by weight ethylhexylglycerin and about 0.25% by weight caprylyl glycol.

In any of the sunscreen compositions described herein, the sunscreen composition may be obtained by a method involving (i) adding the $TiO_2$ at room temperature (i.e., without heating), and then (ii) adding the ZnO.

In a further aspect, the present invention provides a sunscreen composition comprising: (i) $TiO_2$, (ii) ZnO, (iii) triacontanyl PVP, and (iv) VP/hexadecene copolymer, wherein the composition is an oil-in-water emulsion, and the weight ratio of (iii):(iv) is less than one. In some embodiments, the composition is an oil-in-water emulsion substantially free of low molecular weight, organic UV absorbers. In some embodiments, the composition provides an in vitro SPF as measured by the IN VITRO SPF TEST METHOD of at least 25.

In some embodiments of this further aspect, the sunscreen composition comprises less than 25% by weight based on the total weight of the sunscreen composition of the combination of titanium dioxide and zinc oxide.

In some embodiments of this further aspect, the sunscreen composition comprises about 8% by weight $TiO_2$ and about 15% by weight ZnO.

In some embodiments of this further aspect, the weight ratio of (iii):(iv) is less than 0.60.

In some embodiments of this further aspect, the weight ratio of (iii):(iv) is about 0.50.

In some embodiments of this further aspect, the sunscreen composition further comprises about 5% by weight glycerin.

In some embodiments of this further aspect, the sunscreen composition further comprises steareth-21, cetearyl olivate and sorbitan olivate.

In some embodiments of this further aspect, the sunscreen composition has a shear viscosity of less than 32 Pa.s at 1 s$^{-1}$ at 25° C.

In some embodiments of this further aspect, the sunscreen composition further comprises phenoxyethanol, chlorphenesin, ethylhexylglycerin and caprylyl glycol.

In some embodiments of this further aspect, the sunscreen composition comprises about 0.5 to about 2.25% by weight of triacontanyl PVP, preferably about 0.8 to about 1.5% by weight of triacontanyl PVP. For example, the composition comprises about 1% (such as about 1.0%) by weight of triacontanyl PVP.

In some embodiments of this further aspect, the sunscreen composition comprises about 0.5 to about 3% by weight of VP/hexadecene copolymer, preferably about 1 to about 2.5% by weight of VP/hexadecene copolymer. For example, the composition comprises about 2% (such as about 2.0%) by weight of VP/hexadecene copolymer.

In some embodiments of this further aspect, the weight ratio of triacontanyl PVP to VP/hexadecene copolymer may be around 0.5. For example, the weight ratio of triacontanyl PVP to VP/hexadecene copolymer may be 0.5.

In some embodiments of this further aspect, the sunscreen composition comprises about 1% by weight triacontanyl PVP and about 2% by weight of VP/hexadecene copolymer.

The skilled person would appreciate that each of the statements, embodiments and claims that are presented herein are also applicable to this further aspect.

The following non-limiting examples further illustrate the invention.

**EXAMPLE 1**

A series of TiO$_2$/ZnO sunscreen compositions free of low molecular weight UV-absorbers were made using the ingredients shown in Table 1. Compositions 3, 6 and 8-19 were according to the invention, and compositions 1, 2, 4, 5, and 7 were comparative. Composition 1 was AVEENO Calm + Restore Daily Moisturizer Mineral Sunscreen SPF 30, commercially available from Johnson & Johnson Consumer Inc.

The compositions were all water-in-oil emulsions. They were prepared generally by mixing an oil phase and a water phase in a manner sufficient to disperse the oil phase substantially homogeneously in the water phase, such that the water phase was continuous and the oil phase was discontinuous. The oil phase ingredients were caprylic/capric triglyceride, ethylhexyl methoxycrylene, dimethicone, polyhydroxystearic acid, titanium dioxide, aluminum hydroxide, stearic acid, zinc oxide, triethoxycaprylylsilane, cetearyl olivate, sorbitan olivate, calcium sodium borosilicate, steareth-21, triacontanyl PVP, and butyloctyl salicylate. The water phase ingredients were water, xanthan gum, disodium EDTA, sodium chloride, chlorphenesin, caprylyl glycol, phenoxyethanol, ethylhexylglycerin, glycerin, and tocopheryl acetate.

In vitro SPF was tested on the compositions using the IN VITRO SPF TEST METHOD set forth above. The results are also shown in Tables 1, 2 and 3.

The shear viscosity of several of the compositions was also measured using the SHEAR VISCOSITY TEST METHOD and the results are shown in the Tables.

The results show that only compositions according to the invention containing TiO$_2$, ZnO, triacontanyl PVP, and VP/hexadecene copolymer, wherein the weight ratio of triacontanyl PVP to VP/hexadecene copolymer is less than one provided SPF measurements of at least 25 when measured by the IN VITRO SPF TEST METHOD. In addition, these compositions all had shear viscosities less than 32 Pa·s, indicating a desirable, light weight skin feel.

**TABLE 1**

| Ingredient | Composition | | |
|---|---|---|---|
| | 1 | 2 | 3 |
| Butyloctyl Salicylate | 0.00 | 0.00 | 4.00 |
| Phenoxyethanol | 0.50 | 0.00 | 0.60 |
| Cetearyl Alcohol | 0.50 | 0.50 | 0.00 |
| Disodium EDTA | 0.20 | 0.20 | 0.20 |
| Tocopheryl Acetate | 0.20 | 0.20 | 0.20 |
| VP/Hexadecene Copolymer | 1.00 | 1.00 | 2.00 |
| Sodium Chloride | 0.50 | 0.50 | 0.50 |
| Water | 47.45 | 47.45 | 41.78 |
| Phenoxyethanol; Ethylhexylglycerin | 0.00 | 0.56 | 0.00 |
| Water; Glycerin; Avena Sativa (Oat) Kernel Extract | 0.50 | 0.50 | 0.00 |
| Ethylhexylglycerin | 0.50 | 0.45 | 0.50 |
| Titanium Dioxide; Aluminum Hydroxide; Stearic Acid | 7.00 | 7.00 | 8.00 |
| Xanthan Gum | 0.70 | 0.70 | 0.50 |
| Cetearyl Olivate; Sorbitan Olivate | 1.00 | 1.00 | 1.00 |

(continued)

| Ingredient | Composition | | |
|---|---|---|---|
| | 1 | 2 | 3 |
| Triacontanyl PVP | 1.50 | 1.50 | 1.00 |
| Chlorphenesin | 0.25 | 0.25 | 0.27 |
| Glycerin | 4.75 | 4.75 | 5.00 |
| Ethylhexyl methoxycrylene | 3.00 | 3.00 | 3.00 |
| Chrysanthemum Parthenium (Feverfew) Flower/Leaf/Stem Juice | 0.30 | 0.30 | 0.00 |
| Caprylic/Capric Triglyceride | 11.00 | 11.00 | 11.00 |
| Zinc Oxide; Triethoxycaprylylsilane | 13.00 | 13.00 | 15.00 |
| Polyhydroxystearic Acid | 1.00 | 1.00 | 1.00 |
| Caprylyl Glycol | 0.00 | 0.00 | 0.25 |
| Steareth-21 | 0.00 | 0.00 | 1.20 |
| PEG-100 Stearate; Glyceryl Stearate | 2.00 | 2.00 | 0.00 |
| Dimethicone | 2.00 | 2.00 | 2.00 |
| Iron Oxides; Triethoxycaprylylsilane | 0.13 | 0.13 | 0.00 |
| Iron Oxides; Iron Oxides; Triethoxycaprylylsilane | 0.02 | 0.02 | 0.00 |
| Calcium Sodium Borosilicate | 1.00 | 1.00 | 1.00 |
| **Total** | **100.00** | **100.00** | **100.00** |
| | | | |
| **In Vitro SPF** | **18.6** | **18.6** | **34.3** |
| **Shear Viscosity (Pa·s)** | **67.01** | **67.01** | **17.03** |

### TABLE 2

| Ingredient | Composition | | | |
|---|---|---|---|---|
| | 4 | 5 | 6 | 7 |
| Sodium Chloride | 0.50 | 0.50 | 0.50 | 0.50 |
| Triacontanyl PVP | 0.00 | 0.00 | 1.00 | 0.00 |
| Polyhydroxystearic Acid | 1.00 | 1.00 | 1.00 | 1.00 |
| VP/Hexadecene Copolymer | 2.00 | 3.00 | 2.00 | 2.00 |
| Dimethicone | 2.00 | 2.00 | 2.00 | 2.00 |
| Calcium Sodium Borosilicate | 1.50 | 1.50 | 1.50 | 1.50 |
| Titanium Dioxide; Aluminum Hydroxide; Stearic Acid | 7.00 | 7.00 | 7.00 | 7.00 |
| Zinc Oxide; Triethoxycaprylylsilane | 13.00 | 13.00 | 13.00 | 13.00 |
| Glycerin | 5.00 | 5.00 | 5.00 | 5.00 |
| Chlorphenesin | 0.25 | 0.25 | 0.25 | 0.25 |
| Cetearyl Olivate; Sorbitan Olivate | 1.00 | 1.00 | 1.00 | 1.00 |
| Phenoxyethanol | 0.50 | 0.50 | 0.50 | 0.50 |
| Water | 49.65 | 48.65 | 48.65 | 47.65 |
| Caprylic/Capric Triglyceride | 11.00 | 11.00 | 11.00 | 11.00 |
| Ethylhexylglycerin | 0.50 | 0.50 | 0.50 | 0.50 |

(continued)

| Ingredient | Composition | | | |
|---|---|---|---|---|
| | 4 | 5 | 6 | 7 |
| Ethylhexyl methoxycrylene | 3.00 | 3.00 | 3.00 | 3.00 |
| Xanthan Gum | 0.50 | 0.50 | 0.50 | 0.50 |
| Disodium EDTA | 0.20 | 0.20 | 0.20 | 0.20 |
| Tocopheryl Acetate | 0.20 | 0.20 | 0.20 | 0.20 |
| Steareth-21 | 1.20 | 1.20 | 1.20 | 1.20 |
| Acrylates/C12-22 Alkyl Methacrylate Copolymer | 0.00 | 0.00 | 0.00 | 2.00 |
| **Total** | **100.00** | **100.00** | **100.00** | **100.00** |
| | | | | |
| **In Vitro SPF** | **12.00** | **15.20** | **26.90** | **18.70** |
| **Shear Viscosity (Pa·s)** | **18.76** | **14.04** | **22.396** | **21.47** |

**TABLE 3**

| Ingredient | Composition | | | | | | | | | | | |
|---|---|---|---|---|---|---|---|---|---|---|---|---|
| | 8 | 9 | 10 | 11 | 12 | 13 | 14 | 15 | 16 | 17 | 18 | 19 |
| Sodium Chloride | 0.50 | 0.50 | 0.50 | 0.50 | 0.50 | 0.50 | 0.50 | 0.50 | 0.50 | 0.50 | 0.5 | 0.5 |
| Triacontanyl PVP | 1.00 | 1.00 | 1.00 | 1.00 | 1.00 | 1.00 | 1.00 | 1.00 | 1.00 | 1.00 | 1.00 | 1.00 |
| Polyhydroxystearic Acid | 1.00 | 1.00 | 1.00 | 1.00 | 1.00 | 0.00 | 1.00 | 1.00 | 1.00 | 1.00 | 1.00 | 1.00 |
| PEG-100 Stearate; Glyceryl Stearate | 0.00 | 0.00 | 0.00 | 0.00 | 2.00 | 0.00 | 0.00 | 0.00 | 0.00 | 0.00 | 0.00 | 0.00 |
| VP/Hexadecene Copolymer | 2.00 | 2.00 | 2.00 | 2.00 | 2.00 | 2.00 | 2.00 | 2.00 | 2.00 | 2.00 | 2.00 | 2.00 |
| Dimethicone | 2.00 | 2.00 | 2.00 | 2.00 | 2.00 | 2.00 | 2.00 | 2.00 | 0.00 | 2.00 | 2.00 | 2.00 |
| Calcium Sodium Borosilicate | 1.50 | 1.00 | 1.00 | 1.00 | 1.00 | 1.00 | 1.00 | 1.00 | 1.00 | 1.00 | 1.00 | 1.00 |
| Titanium Dioxide; Aluminum Hydroxide; Stearic Acid | 7.00 | 7.00 | 7.00 | 7.00 | 7.00 | 7.00 | 7.00 | 7.00 | 7.00 | 8.00 | 8.00 | 8.00 |
| Zinc Oxide; Triethoxycaprylylsilane | 15.00 | 13.00 | 15.00 | 13.00 | 13.00 | 13.00 | 13.00 | 13.00 | 13.00 | 15.00 | 15.00 | 15.00 |
| Glycerin | 5.00 | 5.00 | 5.00 | 5.00 | 5.00 | 5.00 | 5.00 | 5.00 | 5.00 | 5.00 | 5.00 | 5.00 |
| Chlorphenesin | 0.25 | 0.25 | 0.25 | 0.25 | 0.25 | 0.25 | 0.25 | 0.25 | 0.25 | 0.25 | 0.27 | 0.27 |
| Cetearyl Olivate; Sorbitan Olivate | 1.00 | 1.00 | 1.00 | 1.00 | 1.00 | 1.00 | 1.00 | 1.00 | 1.00 | 1.00 | 1.00 | 1.00 |
| Phenoxyethanol | 0.50 | 0.50 | 0.50 | 0.50 | 0.50 | 0.50 | 0.50 | 0.50 | 0.50 | 0.50 | 0.50 | 0.60 |
| Water | 46.65 | 49.15 | 47.15 | 48.65 | 47.85 | 50.15 | 48.65 | 48.65 | 49.15 | 42.15 | 42.13 | 41.78 |
| Caprylic/Capric Triglyceride | 11.00 | 11.00 | 11.00 | 11.00 | 11.00 | 11.00 | 11.00 | 11.00 | 11.00 | 11.00 | 11.00 | 11.00 |
| Ethylhexylglycerin | 0.50 | 0.50 | 0.50 | 0.50 | 0.50 | 0.50 | 0.50 | 0.50 | 0.50 | 0.50 | 0.50 | 0.50 |
| Ethylhexyl methoxycrylene | 3.00 | 3.00 | 3.00 | 3.00 | 3.00 | 3.00 | 3.00 | 3.00 | 3.00 | 3.00 | 3.00 | 3.00 |
| Cetearyl Alcohol | 0.00 | 0.00 | 0.00 | 0.50 | 0.50 | 0.00 | 0.00 | 0.00 | 0.00 | 0.00 | 0.00 | 0.00 |
| Xanthan Gum | 0.50 | 0.50 | 0.50 | 0.50 | 0.50 | 0.50 | 0.50 | 0.50 | 0.50 | 0.50 | 0.50 | 0.50 |
| Disodium EDTA | 0.20 | 0.20 | 0.20 | 0.20 | 0.20 | 0.20 | 0.20 | 0.20 | 0.20 | 0.20 | 0.20 | 0.20 |
| Tocopheryl Acetate | 0.20 | 0.20 | 0.20 | 0.20 | 0.20 | 0.20 | 0.20 | 0.20 | 0.20 | 0.20 | 0.20 | 0.20 |
| Steareth-21 | 1.20 | 1.20 | 1.20 | 1.20 | 0.00 | 1.20 | 1.20 | 1.20 | 1.20 | 1.20 | 1.20 | 1.20 |
| Acrylates/C12-22 Alkyl Methacrylate Copolymer | 0.00 | 0.00 | 0.00 | 0.00 | 0.00 | 0.00 | 0.50 | 0.50 | 0.00 | 0.00 | 0.00 | 0.00 |
| SP Arlamol PS15E-MBAL-LQ-(AP) | 0.00 | 0.00 | 0.00 | 0.00 | 0.00 | 0.00 | 0.00 | 0.00 | 2.00 | 0.00 | 0.00 | 0.00 |
| Butyloctyl Salicylate | 0.00 | 0.00 | 0.00 | 0.00 | 0.00 | 0.00 | 0.00 | 0.00 | 0.00 | 4.00 | 4.00 | 4.00 |

| Ingredient | Composition | | | | | | | | | | | |
|---|---|---|---|---|---|---|---|---|---|---|---|---|
| | 8 | 9 | 10 | 11 | 12 | 13 | 14 | 15 | 16 | 17 | 18 | 19 |
| Caprylyl Glycol | 0.00 | 0.00 | 0.00 | 0.00 | 0.00 | 0.00 | 0.00 | 0.00 | 0.00 | 0.00 | 0.00 | 0.25 |
| **Total** | **100** | **100** | **100** | **100** | **100** | **100** | **100** | **100** | **100** | **100** | **100** | **100** |
| | | | | | | | | | | | | |
| **In Vitro SPF** | **31.70** | **28.60** | **28.66** | **31.02** | **27.53** | **25.10** | **29.50** | **25.60** | **25.80** | **34.30** | **28.83** | **27.17** |
| **Shear Viscosity (Pa·s)** | **15.34** | **17.03** | | | | | | | | | **27.25** | |

EP 4 684 774 A1

**EXAMPLE 2**

Compositions 1 and 3 were tested in vivo for SPF using the methodology set forth in the US FDA OTC monograph for sunscreens (U.S. Food and Drug Administration Over-the-Counter Monograph M020: Sunscreen Drug Products for Over-the-Counter Human Use (Posted September 24, 2021). Final Administrative Order (OTC000006), effective upon enactment of the Coronavirus Aid, Relief, and Economic Security Act (CARES Act), Public Law 116-136, on March 27, 2020).

Composition 3 according to the invention provided an in vivo SPF of 70, while Composition 1 provided an in vivo SPF of only 33. These results corroborate the unexpectedly high photoprotective activity of compositions according to the invention containing triacontanyl PVP and VP/hexadecene copolymer, wherein the weight ratio of triacontanyl PVP to VP/hexadecene copolymer is less than 1. Compositions 1 and 3 both contained triacontanyl PVP and VP/hexadecene copolymer. However, the weight ratio of the film formers in Composition 1 was 1.5, while the weight ratio of the film formers in Composition 3 was 0.5.

**Exemplary embodiments:**

1. A sunscreen composition comprising: (i) $TiO_2$, (ii) ZnO, (iii) triacontanyl PVP, and (iv) VP/hexadecene copolymer, wherein the composition is an oil-in-water emulsion substantially free of low molecular weight, organic UV absorbers, the weight ratio of (iii):(iv) is less than one, and the composition provides an in vitro SPF as measured by the IN VITRO SPF TEST METHOD of at least 25.

2. The sunscreen composition of embodiment 1 comprising less than 25% by weight based on the total weight of the sunscreen composition of the combination of titanium dioxide and zinc oxide.

3. The sunscreen composition of embodiment 1 comprising about 8% by weight $TiO_2$ and about 15% by weight ZnO.

4. The sunscreen composition of embodiment 1, wherein the weight ratio of (iii):(iv) is less than 0.60.

5. The sunscreen composition of embodiment 1, wherein the weight ratio of (iii):(iv) is about 0.50.

6. The sunscreen composition of embodiment 1 further comprising about 5% by weight glycerin.

7. The sunscreen composition of embodiment 1 further comprising steareth-21, cetearyl olivate and sorbitan olivate.

8. The sunscreen composition of embodiment 1 having a shear viscosity of less than 32 Pa.s at 1 s$^{-1}$ at 25° C.

9. The sunscreen composition of embodiment 1 further comprising phenoxyethanol, chlorphenesin, ethylhexylglycerin and caprylyl glycol.

10. A sunscreen composition comprising: (i) $TiO_2$, (ii) ZnO, (iii) triacontanyl PVP, and (iv) VP/hexadecene copolymer, wherein the composition is an oil-in-water emulsion, and the weight ratio of (iii):(iv) is less than one.

11. The sunscreen composition of embodiment 10 wherein the composition is an oil-in-water emulsion substantially free of low molecular weight, organic UV absorbers.

12. The sunscreen composition of embodiment 10 or 11 comprising less than 25% by weight based on the total weight of the sunscreen composition of the combination of titanium dioxide and zinc oxide.

13. The sunscreen composition of any one of embodiments 10-12 comprising about 8% by weight $TiO_2$ and about 15% by weight ZnO.

14. The sunscreen composition of any one of embodiments 10-12 comprising about 7% by weight $TiO_2$ and/or about 14% by weight ZnO, preferably about 6.64% by weight $TiO_2$ and about 14.4% by weight ZnO.

15. The sunscreen composition of any one of embodiments 10-14, wherein the weight ratio of (iii):(iv) is less than 0.60.

16. The sunscreen composition of any one of embodiments 10-15, wherein the weight ratio of (iii):(iv) is about 0.50.

17. The sunscreen composition of any one of embodiments 10-16 further comprising about 5% by weight glycerin.

18. The sunscreen composition of any one of embodiments 10-17 further comprising steareth-21, cetearyl olivate and sorbitan olivate.

19. The sunscreen composition of any one of embodiments 10-18 having a shear viscosity of less than 32 Pa.s at 1 s$^{-1}$ at 25° C.

20. The sunscreen composition of any one of embodiments 10-19 further comprising phenoxyethanol, chlorphenesin, ethylhexylglycerin and caprylyl glycol.

21. The sunscreen composition of any one of embodiments 10-20, wherein the composition comprises about 0.5 to about 2.25% by weight of triacontanyl PVP, preferably about 0.8 to about 1.5% by weight of triacontanyl PVP.

22. The sunscreen composition of any one of embodiments 10-21, wherein the composition comprises about 1% by weight of triacontanyl PVP.

23. The sunscreen composition of any one of embodiments 10-22, wherein the composition comprises about 0.5 to about 3% by weight of VP/hexadecene copolymer, preferably about 1 to about 2.5% by weight of VP/hexadecene copolymer.

24. The sunscreen composition of any one of embodiments 10-23, wherein the composition comprises about 2% by weight of VP/hexadecene copolymer.

25. The sunscreen composition of any one of embodiments 10-24, wherein the composition comprises about 1% by weight triacontanyl PVP and about 2% by weight of VP/hexadecene copolymer.

**Claims**

1. A sunscreen composition comprising: (i) TiO$_2$, (ii) ZnO, (iii) triacontanyl PVP, and (iv) VP/hexadecene copolymer, wherein the composition is an oil-in-water emulsion substantially free of low molecular weight, organic UV absorbers, the weight ratio of (iii):(iv) is less than one, and the composition provides an in vitro SPF as measured by the **IN** VITRO SPF TEST METHOD of at least 25.

2. The sunscreen composition of claim 1 comprising less than 25% by weight based on the total weight of the sunscreen composition of the combination of titanium dioxide and zinc oxide.

3. The sunscreen composition of claim 1 or claim 2 comprising about 8% by weight TiO$_2$ and about 15% by weight ZnO.

4. The sunscreen composition of claim 1 or claim 2 comprising about 7% by weight TiO$_2$ and/or about 14% by weight ZnO, preferably about 6.64% by weight TiO$_2$ and about 14.4% by weight ZnO.

5. The sunscreen composition of any one of claims 1-4, wherein the weight ratio of (iii):(iv) is less than 0.60.

6. The sunscreen composition of any one of claims 1-5, wherein the weight ratio of (iii):(iv) is about 0.50.

7. The sunscreen composition of any one of claims 1-6 further comprising about 5% by weight glycerin.

8. The sunscreen composition of any one of claims 1-7 further comprising steareth-21, cetearyl olivate and sorbitan olivate.

9. The sunscreen composition of any one of claims 1-8 having a shear viscosity of less than 32 Pa.s at 1 s$^{-1}$ at 25° C.

10. The sunscreen composition of any one of claims 1-9 further comprising phenoxyethanol, chlorphenesin, ethylhexylglycerin and caprylyl glycol.

11. The sunscreen composition of any one of claims 1-10, wherein the composition comprises about 0.5 to about 2.25% by weight of triacontanyl PVP, preferably about 0.8 to about 1.5% by weight of triacontanyl PVP.

12. The sunscreen composition of any one of claims 1-11, wherein the composition comprises about 1% by weight of triacontanyl PVP.

13. The sunscreen composition of any one of claims 1-12, wherein the composition comprises about 0.5 to about 3% by weight of VP/hexadecene copolymer, preferably about 1 to about 2.5% by weight of VP/hexadecene copolymer.

14. The sunscreen composition of any one of claims 1-13, wherein the composition comprises about 2% by weight of VP/hexadecene copolymer.

15. The sunscreen composition of any one of claims 1-14, wherein the composition comprises about 1% by weight triacontanyl PVP and about 2% by weight of VP/hexadecene copolymer.

Europäisches
Patentamt

European
Patent Office

Office européen
des brevets

## EUROPEAN SEARCH REPORT

Application Number

EP 25 19 1654

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DATABASE GNPD [Online] MINTEL; 17 April 2024 (2024-04-17), anonymous: "Daily Moisturizer SPF 30", XP093346068, Database accession no. 11640926 * the whole document * | 1-15 | INV. A61K8/27 A61K8/29 A61K8/49 A61K8/81 A61Q17/04 |
| Y | US 2007/218021 A1 (WELLS RICHARD [US]) 20 September 2007 (2007-09-20) * claims 1-4,7,23 * * paragraphs [0019] - [0028], [0007], [0017] * | 1-15 | |
| Y | US 2024/058237 A1 (STAHL CHRISTOPHER RYAN [US] ET AL) 22 February 2024 (2024-02-22) * claims 1,2,14 * * paragraphs [0034], [0036] * * paragraphs [0103], [0123]; tables 1,4,5,8 * | 1-15 | |
| Y | SHEILA M. DELL ET AL: "Avicel RC/CL, Microcrystalline Cellulose and Carboxymethylcellulose Sodium, NF, BP", FMC CORPORATION, 1 January 2001 (2001-01-01), pages 1-28, XP055738286, * left column; page 23 * | 1-15 | **TECHNICAL FIELDS SEARCHED (IPC)** A61K A61Q |
| Y | DATABASE GNPD [Online] MINTEL; 27 September 2017 (2017-09-27), anonymous: "Sun Milk SPF 50", XP093346167, Database accession no. 5127265 * the whole document * | 1-15 | |

-/--

The present search report has been drawn up for all claims

| Place of search | Date of completion of the search | Examiner |
|---|---|---|
| Munich | 12 December 2025 | Grenouillat, N |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

Europäisches
Patentamt

European
Patent Office

Office européen
des brevets

# EUROPEAN SEARCH REPORT

**Application Number**

EP 25 19 1654

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2010/278882 A1 (LIEBMANN BURGHARD [DE] ET AL) 4 November 2010 (2010-11-04) * composition 1; pages 31-32 * ----- | 1-15 | |
| | | | **TECHNICAL FIELDS SEARCHED (IPC)** |

The present search report has been drawn up for all claims

| Place of search | Date of completion of the search | Examiner |
|---|---|---|
| Munich | 12 December 2025 | Grenouillat, N |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 25 19 1654

This annex lists the patent family members relating to the patent documents cited in the above-mentioned European search report.
The members are as contained in the European Patent Office EDP file on
The European Patent Office is in no way liable for these particulars which are merely given for the purpose of information.

12-12-2025

| Patent document cited in search report | Publication date | Patent family member(s) | | Publication date |
|---|---|---|---|---|
| US 2007218021 A1 | 20-09-2007 | NONE | | |
| US 2024058237 A1 | 22-02-2024 | AU | 2023323667 A1 | 30-01-2025 |
| | | CN | 120051268 A | 27-05-2025 |
| | | EP | 4568753 A1 | 18-06-2025 |
| | | JP | 2025526593 A | 15-08-2025 |
| | | KR | 20250058749 A | 30-04-2025 |
| | | US | 2024058237 A1 | 22-02-2024 |
| | | WO | 2024035556 A1 | 15-02-2024 |
| US 2010278882 A1 | 04-11-2010 | CA | 2637065 A1 | 26-07-2007 |
| | | CA | 2638870 A1 | 26-07-2007 |
| | | CN | 101370481 A | 18-02-2009 |
| | | CN | 101370556 A | 18-02-2009 |
| | | CN | 104856962 A | 26-08-2015 |
| | | EP | 1978937 A1 | 15-10-2008 |
| | | EP | 1979055 A2 | 15-10-2008 |
| | | JP | 5236498 B2 | 17-07-2013 |
| | | JP | 2009523766 A | 25-06-2009 |
| | | JP | 2009523767 A | 25-06-2009 |
| | | US | 2010278882 A1 | 04-11-2010 |
| | | US | 2010278883 A1 | 04-11-2010 |
| | | WO | 2007082923 A2 | 26-07-2007 |
| | | WO | 2007082936 A1 | 26-07-2007 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 13645623